Europäisches Patentamt

European Patent Office

Office européen des brevets

(19)

(11) Publication number : **0 389 992 B1**

## (12) EUROPEAN PATENT SPECIFICATION

(45) Date of publication of patent specification :
31.08.94 Bulletin 94/35

(51) Int. Cl.⁵ : **G06F 15/42, G06F 15/00**

(21) Application number : **90105598.8**

(22) Date of filing : **23.03.90**

(54) **Apparatus for automatically analyzing validity of medical examination data.**

(30) Priority : **31.03.89 JP 78610/89**

(43) Date of publication of application :
**03.10.90 Bulletin 90/40**

(45) Publication of the grant of the patent :
**31.08.94 Bulletin 94/35**

(84) Designated Contracting States :
**DE NL**

(56) References cited :
US-A- 4 202 033
IEEE INTERNATIONAL CONFERENCE ON
NEURAL NETWORKS, San Diego, California,
24th -27th July 1988, pages I-255 - I-262, IEEE,
New York, US; K. SAITO et al.:"Medical diag-
nostic expert system based on PDP model"

(56) References cited :
IEEE INTERNATIONAL CONFERENCE ON
NEURAL NETWORKS, San Diego, California,
24th -27th July 1988, pages I-561 - I-568, IEEE,
New York, US; D.D. EGBERT et
al.:"Preprocessing of biomedical images for
neurocomputer analysis"

(73) Proprietor : **KABUSHIKI KAISHA TOSHIBA**
72, Horikawa-cho
Saiwai-ku
Kawasaki-shi Kanagawa-ken Tokyo (JP)

(72) Inventor : **Ohhashi,Akinami**
4-4-5 Sakurada, Washimiyacho
Kitakatsuhikagun, Saitama-ken (JP)

(74) Representative : **Blumbach Weser Bergen
Kramer Zwirner Hoffmann Patentanwälte**
Radeckestrasse 43
D-81245 München (DE)

EP 0 389 992 B1

## Description

The present invention relates to an apparatus for automatically analyzing validity of medical examination data obtained by an examination of an object to be examined, which determines whether re-examination is necessary or not, automatically.

A typical conventional apparatus for automatically analyzing validity of medical examination data has been disclosed in Japanese Patent Application Laid Open No. 62-220868 (1987).

In this conventional apparatus, whether there is any error in the medical examination data is determined by observing a pattern of the medical examination data values for a particular medical examination and comparing this pattern with patterns of the medical examination data values previously obtained for the same particular medical examination, in which the medical examination data are considered erroneous when the pattern of the medical examination data values is infrequently encountered one.

However, the medical examination data having an infrequently encountered pattern are not necessarily erroneous. On the other hand, the medical examination data having a frequently encountered pattern are not always without an error. Thus, the accuracy of the decision of the validity has been rather low in a conventional apparatus, and a conventional apparatus has been practically too unreliable.

IEEE International Conference On Neural Networks, San Diego, California, 24th - 27th of July 1988, pp I-255, to I-262, IEEE, New York, US, discloses the use of a diagnostic expert system based on a multi-layer network to extract knowledge from a patient.

It is therefore an object of the present invention to provide an apparatus for automatically analyzing validity of medical examination data capable of making correctable assessment for a necessity of the re-examination. This object is achieved by the present invention in accordance with the features of claim 1.

Other features and advantages of the present invention will become apparent from the following description taken in conjunction with the accompanying drawings.

## BRIEF DESCRIPTION OF THE DRAWINGS

Fig. 1 is a schematic block diagram of one embodiment of an apparatus for automatically analyzing validity of medical examination data according to the present invention.

Fig. 2 is a block diagram of a neural network utilized in the apparatus of Fig. 1.

## DETAILED DESCRIPTION OF THE PREFERRED EMBODIMENTS

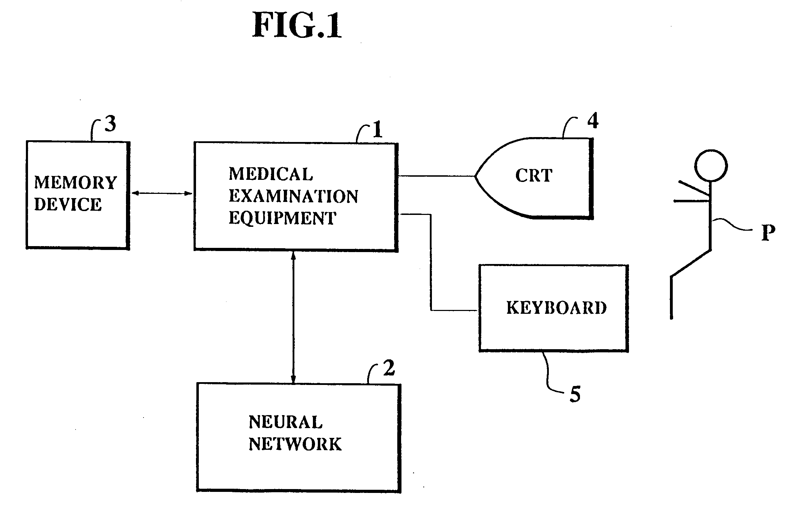

Referring now to Fig. 1, there is shown one embodiment of an apparatus for automatically analyzing validity of medical examination data according to the present invention.

In this embodiment, the apparatus comprises an medical examination equipment 1 for medically examining an object to be examined, processing data obtained from the object to be examined, and producing a result of a medical examination in terms of medical examination data, a neural network 2 for making an assessment for a necessity of re-examination by analyzing validity of the medical examination data and learning previous analyses in order to improve an accuracy of the assessment, a memory device 3 for memorizing the medical examination data and teacher data to accompany the medical examination data, both of which are to be utilized in a process of learning by the neural network 2, a CRT 4 for displaying the assessment made by the neural network 2 for the sake of inspection by an operator P, and a keyboard 5 for allowing the operator P to indicate his judgement for the necessity of the re-examination.

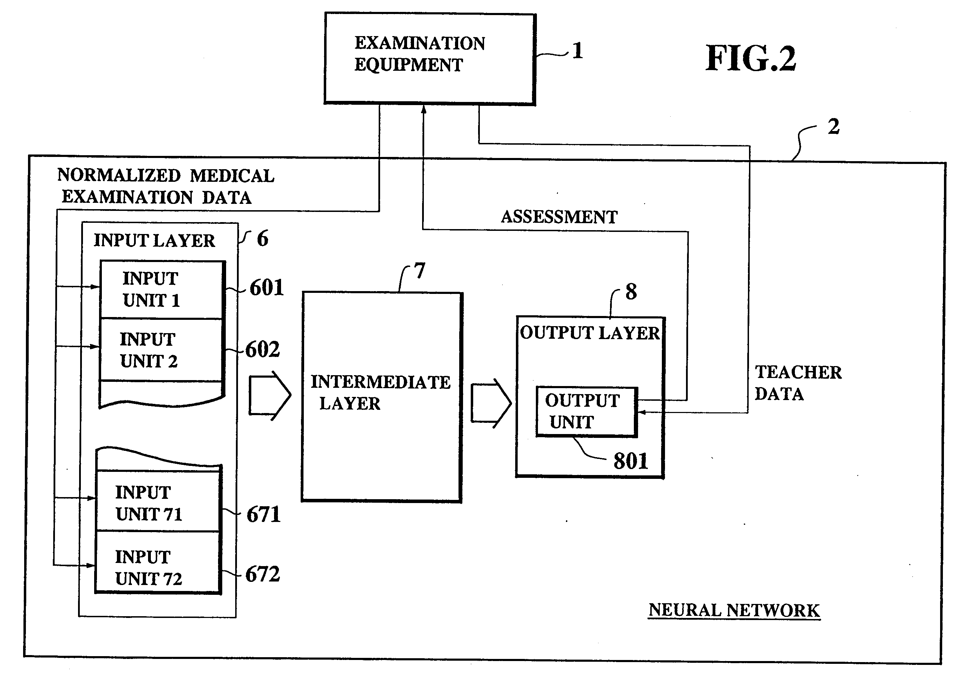

Referring now to Fig. 2, a detail of the neural network 2 in this apparatus will be described. Here, for the sake of explanation, it is assumed that each set of the medical examination data comprises 36 items.

As shown in Fig. 2, the neural network 2 in this apparatus has a triple layer structure comprising an input layer 6, an intermediate layer 7, and an output layer 8, and in this case the input layer 6 is equipped with 72 input units 601-672, while the output layer 8 is equipped with only one output unit 801. The intermediate layer 7 may be equipped with much greater number of units such as 200 units for example. The input units 601-672 are assumed to be those capable of inputting continuously varying values between 0 and 1, while the output units 801 is assumed to be that capable of outputting continuously varying value between 0 and 1. As in a usual neural network, the units of the input, intermediate, and output layers utilize the sigmoid function f(x) given by the expression:

$$f(x) = 1/\{1 + \exp(-x + b)\}$$

where b is a so called bias, which is a weight for those units having their outputs equal to 1, and which is determined in the course of learning.

Now, in this apparatus, in order for the neural network 2 to operate, an initial learning must be carried out on the neural network 2 using an appropriate number of the medical examination data and accompanying teacher data which indicates whether the re-examination is performed or not for these particular medical examination data. This initial learning will be described in detail below.

Assuming that the initial learning of the neural network 2 has already been completed by using an appropriate number of the medical examination data and the teacher data, the apparatus operates as follows.

First of all, when the medical examination data are collected by the medical examination equipment 1, the medical examination equipment 1 normalizes the collected medical examination data to obtain normalized medical examination data within a range between 0 and 1, and supply these normalized medical examination data to the neural network 2.

More specifically, the normalized medical examination data to be given to the neural network 2 are obtained by the medical examination equipment 1 as follows.

The normalization is carried out independently for each item of a set of the medical examination data. Here, any monotonously increasing function may be utilized for the normalization, but it is generally more preferable to have as wide a dynamic range for the normalization as possible. For example, in some cases the medical examination data may differ for a normal person and for a sick person by an order of several hundred. In such a case, it is desirable to use logarithmic function for the normalization. In the following, a case using a linear function for the normalization will be elaborated.

In this embodiment, the normalization is achieved by using the following linear function:

$$C = \frac{(X - B)}{(A - B)} \times (1 - d) + d$$

where X is a value before the normalization, C is a normalized value, A is an expectation value for the maximum, B is an expectation value for the minimum, and d is a coefficient specifying a lower bound. The upper bound is set equal to 1 in this embodiment. Thus, when the coefficient d is set equal to 0.2, all the normalized values fall within a range between 0.2 and 1.

In addition to calculating the normalized medical examination data as described above, the medical examination equipment 1 also generates additional data indicating the presence or absence of each item of a set of the medical examination data. Namely, certain items of a set of the medical examination data may be missing because they were not collected as being unnecessary for that particular examination. Thus, for 36 items of a set of the medical examination data in this embodiment, the medical examination equipment 1 generates 36 additional data, each of which is given a value of either 1 indicating the presence of the medical examination data for a particular item, or 0 indicating the absence of the medical examination data for a particular item. Such additional data provide information required by the neural network 2 in arriving at correct assessment, as the assessment to be made relies on which items of a set of the medical examination data have been measured.

The input units 601-672 of the neural network 2 are provided in correspondence with these 36 items of the medical examination data and 36 additional data, such that the first 36 input units 601-636 receive 36 medical examination data of 36 different items, while the second 36 input units 637-372 receive 36 additional data for 36 different items.

When these medical examination data and the additional data are supplied to the input units 601-672 of the neural network 2 as input data, the neural network 2 operates on these input data, makes the assessment for the necessity of re-examination in accordance with human judgements learned at the initial learning, and returns the assessment to the examination equipment 1. Here, the assessment is given in a form of a number between 0 and 1, where 0 indicates that the re-examination is absolutely not necessary, while 1 indicates that the re-examination is absolutely necessary.

This assessment is either shown directly to the operator P through the CRT 4 for his inspection, or else further final judgement is derived from this assessment at the medical examination equipment 1 in such a manner for example that the re-examination is judged necessary when the assessment from the neural network 2 falls within a range of 0.7 to 1, whereas the re-examination is judged unnecessary when the assessment from the neural network 2 falls within a range of 0 to 0.3, and the judgement is indeterminate when the assessment from the neural network 2 falls within a range of 0.3 to 0.7 in which case the judgement is entrusted to the discretion of the operator P.

In either case, when the displayed assessment from the neural network 2 is in agreement with the operator's own judgement for a necessity of re-examination, the operator P is required to do nothing and this assessment made by the neural network 2 will be taken as a final judgement, whereas when the displayed assessment from the neural network 2 is not in agreement with the operator's own judgement for a necessity of re-examination, the operator P indicates his judgement as a final judgement through the keyboard 5.

Then, when the final judgement thus determined indicates that the re-examination is necessary, the operator P operates the apparatus to perform the re-examination.

3

Next, the learning of the neural network 2 will be described.

In this embodiment, the learning is performed by the backward error propagation algorithm of Rumelhart, and the learning process is iterated until an error becomes lower than a prescribed level.

Thus, for example, 100,000 sets of the medical examination data and the corresponding teacher data may be collected and compiled in the memory device 3, memorized in an order of acquisition of these medical examination data.

Then, the normalized medical examination data and the corresponding additional data are calculated for each set of the medical examination data by the medical examination equipment 1 as described above, and are given to the input units 601-672 of the neural network 2, while the accompanying teacher data for this set of the medical examination data are given to the output unit 801 of the neural network 2. Then, the learning according to the backward error propagation algorithm is carried out. This process is repeated for all of 100,000 sets of the medical examination data and accompanying teacher data, and is iterated until an error become lower than a prescribed level. Here, the neural network 2 utilizes only a single set of weights.

In this apparatus, the memory device 3 can be utilized for the purpose of re-learning as follows.

Namely, each time the assessment made by the neural network 2 disagrees with the judgement of the operator P for a certain set of the medical examination data, the teacher data accompanying these medical examination data are modified in accordance with the final judgement given by the operator P, and the medical examination data and the modified teacher data for the neural network 2 are memorized in the memory device 3. Alternatively, such a memorization of the medical examination data and the teacher data may be carried out selectively for only a predetermined number of the medical examination data and the teacher data.

In the memory device 3, as many of the medical examination data and the corresponding teacher data as a full storage capacity of the memory device 3 are initially compiled, as the learning data for the initial learning of the neural network 2, which are arranged in an order of acquisition of these medical examination data. When the assessment made by the neural network 2 is corrected at the keyboard 5 by the operator P, the oldest of the medical examination data and the corresponding teacher data memorized in the memory device 3 are released from the memory device 3 and the medical examination data and the corresponding teacher data with respect to which the assessment made by the neural network 2 has been corrected are newly memorized in the memory device 3.

When a number of newly memorized medical examination data and corresponding teacher data reaches a predetermined number, a re-learning of the neural network 2 will be carried out. The re-learning of the neural network 2 may also be carried out when the previous learning is considered insufficient, when the learning data at the previous learning are considered biased, when the configuration of the medical examination equipment 1 is modified, or when the examination to be performed is modified.

Thus, according to this embodiment, it is possible to provide an apparatus for automatically analyzing validity of medical examination data capable of making accurate assessment for the necessity of the re-examination, reflecting the characteristics of each examination apparatus, examination itself, and operator. As a consequence, it is possible according to the present invention to improve the accuracy of the assessment for the necessity of the re-examination. Furthermore, according to the present invention, the accuracy of the assessment for the necessity of the re-examination can further be improved through the learning of the neural network, even when the operational circumstances are changed.

It is to be noted that a concept of groups (or patterns) for combinations of items of the medical examination data may be introduced in the above embodiment as follows.

Namely, for 36 items of the medical examination data, there are as many as $2^{36}$ distinct combinations of the items, but in practice this number may be lowered by discarding inconsistent or unnecessary combinations according to the purpose of the medical examinations to be performed. The distinct combinations of the items to be utilized will be referred to as groups (or patterns). Each group can be devised very freely by simply combining as many as necessary number of items. The same item may belong to more than one group, and one group may entirely overlap with a part of another group. All the combination not to be utilized may be considered as forming one group.

Thus, the medical examination data and the learning data are classified into groups, and the groups are memorized separately in the memory device 3. When a particular item of a particular group has not been collected, this particular item is considered missing. When a particular item of the medical examination data belonging to more than one group is collected, this particular item may be considered as a member of one group to which it belongs and which has fewest missing members. The learning as well as assessment making by the neural network 2 are also carried out for each group separately. Thus, the neural network 2 utilizes different sets of weights for different groups. When a number of newly memorized medical examination data and corresponding teacher data reaches a predetermined number within a particular group, a re-learning of the neural network 2 will be carried out for such a particular group. In addition, a plurality of independent neural networks may be provided for each distinctive group.

The use of groups as described above has an advantage that a much faster learning of the neural network 2 becomes possible because each group concerns only with limited number of items which is much smaller than a total number of items which the apparatus as a whole have to deal with.

It is also to be noted that the input units 601-672 of the neural network 2 may be modified as follows, when a very wide dynamic range for the normalization is required.

Namely, in such a case, a compression of the medical examination data into values within a range between 0 and 1 may cause a poor accuracy for the medical examination data. To avoid this situation, more than one input units of the neural network 2 may be assigned to a single item of a set of the medical examination data. For example, a range for values of a particular item of a set of the medical examination data may be divided into the following three sections:

$B \leq X < A1$      normal
$A1 \leq X < A2$      large
$A2 \leq X < A$      very large

and three input units R, S, and T may be assigned for each of these sections, respectively. In this case, the normalization may be carried out as follows. For normal values of $B \leq X < A1$, the normalization can be achieved by using the following linear function:

$$Cr = \frac{(X - B)}{(A1 - B)} \times (1 - d) + d$$

where X is a value before the normalization, Cr is a normalized value for the input unit R, A1 is a border between the normal and large sections, B is an expectation value for the minimum, and d is a coefficient specifying a lower bound. The upper bound is set equal to 1 as before. When the data is in this range, the input units S and T are given a value of 0. For large values of $A1 \leq X < A2$, the normalization can be achieved by using the following linear function:

$$Cs = \frac{(X - A1)}{(A2 - A1)} \times (1 - d) + d$$

where X is a value before the normalization, Cs is a normalized value for the input unit S, A1 is a border between the normal and large sections, A2 is a border between the large and very large sections, and d is a coefficient specifying a lower bound. The upper bound is set equal to 1 as before. When the data is in this range, the input units R and T are given a value of 0. For very large values of $A2 \leq X < A$, the normalization can be achieved by using the following linear function:

$$Ct = \frac{(X - A2)}{(A - A2)} \times (1 - d) + d$$

where X is a value before the normalization, Ct is a normalized value for the input unit T, A is an expectation value for the maximum, A2 is a border between the large and very large sections, and d is a coefficient specifying a lower bound. The upper bound is set equal to 1 as before. When the data is in this range, the input units R and S are given a value of 0.

It should be noted here that a number of input units to be assigned to an item may vary from one item to another. Also, a formula for the normalization may be different for different input units assigned to the same item. For example, the normal values can be treated by the linear function as described above, while the very large values are treated by a logarithmic function. It is to be also noted that even when the number of input units for the medical examination data is increased as above, a single input unit is sufficient for each additional data to be generated in conjunction with each item of a set of the medical examination data.

It is also to be noted that each one of the input units 601-672 which are assumed to be those capable of inputting continuously varying values between 0 and 1, and the output units 801 which is assumed to be that capable of outputting continuously varying value between 0 and 1 may be replaced by a plurality of units capable of dealing only with discrete values which are assigned to different ranges of data values for each medical examination data. For example, a data value for each medical examination data may be divided into five ranges, such as 0 to 0.2, 0.2 to 0.4, 0.4 to 0.6, 0.6 to 0.8, 0.8 to 1, and five units may be assigned to each of such a range to indicate which one of these ranges does the data value fall.

It is further to be noted that a type of the neural network and a learning algorithm described above may be replaced by other known type of the neural network and the learning algorithm so long as the substantially the same effect as described above is realizable by them.

## Claims

1.     An apparatus for automatically analysing validity of medical examination data by making an assessment of a necessity of re-examination, comprising:

means (1) for collecting and examining the medical examination data and for generating additional data indicating the presence or absence of each item of a set of the medical examination data;

neural network (2)

for learning human judgements of the necessity of re-examination from previously collected sets of medical examination data accompanied with corresponding teacher data, said teacher data indicating whether re-examination was necessary for the corresponding sets of medical examination data whereby the learning by means of the backward error propagation algorithm is repeated until an error becomes lower than a prescribed level; and after this initial learning

for providing an assessment for the necessity of re-examination for each set of the medical examination data collected by the collecting means; and

means for correcting the assessment for the necessity of re-examination made by the neutral network (2) if it is considered insufficient in comparison with human judgement by modifying the teacher data and subsequently re-learning.

2.  The apparatus of claim 1, wherein the neural network comprises means for normalizing said medical examination data so as to obtain data having varying values between 0 and 1.

3.  The apparatus of claim 1, wherein each value of the medical examination data is divided into a plurality of distinct ranges and the neural network (2) is equipped with a plurality of input units (601 - 672) capable of dealing with discrete values which are assigned to the distinct ranges for each value of the medical examination data, the plurality of input units (601 - 672) indicating in which one of the distinct ranges does each value of the medical examination data fall.

4.  The apparatus of claim 1, further comprising:

means (3) for memorizing the medical examination data and the corresponding teacher data each time the assessment made by the neural network is corrected at the correcting means,

and wherein the neural network (2) learns the human judgements for the necessity of re-examination again after new medical examination data and corresponding teacher data are newly memorized in the memorizing means (3).

5.  The apparatus of claim 4, wherein the memorizing means memorizes the medical examination data and the corresponding teacher data in an order of acquisition of the medical examination data, and wherein newest medical examination data and corresponding teacher data with respect to which the assessment made by the neural network (2) is corrected replace oldest medical examination data and corresponding teacher data in the memorizing means (3).

6.  The apparatus of claim 1, wherein the medical examination data comprises a plurality of items, and the medical examination data are classified into groups, each group representing a distinct combination of an arbitrary number of the items, and wherein the learning and assessment making by the neural network are carried out with respect to each group separately.

7.  The apparatus of claim 2, wherein each value of the medical examination data is divided into a plurality of distinct ranges, the neural network (2) is equipped with a plurality of input units (601 - 672) capable of dealing with continuously varying values which are assigned to the distinct ranges for each value of the medical examination data, and the normalization is carried out with respect to each one of the distinct ranges independently.

**Patentansprüche**

1.  Vorrichtung zum automatischen Analysieren der Gültigkeit medizinischer Untersuchungsdaten durch Vornahme einer Abschätzung einer Notwendigkeit für eine Neuuntersuchung, umfassend:

eine Einrichtung (1) zum Sammeln und zum Untersuchen der medizinischen Untersuchungsdaten sowie zum Generieren zusätzlicher Daten, die das Vorhandensein oder das Fehlen jedes Elements eines Satzes der medizinischen Untersuchungsdaten angeben;

ein neurales Netzwerk (2)

zum Lernen menschlicher Beurteilungen der Notwendigkeit einer Neuuntersuchung aus zuvor gesammelten Sätzen medizinischer Untersuchungsdaten, die von entsprechenden Lehrerdaten begleitet werden, wobei die Lehrerdaten angeben, ob für die entsprechenden Sätze medizinischer Untersuchungsdaten eine Neuuntersuchung notwendig war, wodurch das Lernen mit Hilfe des Algorithmus' der Fehlerfortpflanzung in Rückwärtsrichtung solange wiederholt wird, bis der Fehler niedriger als ein vorbestimmter Wert wird, und - nach diesem anfänglichen Lernen -

zum Liefern einer Einschätzung der Notwendigkeit einer Neuuntersuchung für jeden Satz der von der Sammeleinrichtung gesammelten medizinischen Untersuchungsdaten; und

eine Einrichtung zum Korrigieren der Einschätzung der Notwendigkeit einer Neuuntersuchung, die durch das neurale Netzwerk (2) vorgenommen wurde, wenn sie im Vergleich zu der menschlichen Beurteilung als unzulänglich erachtet wird, indem die Lehrerdaten modifiziert und anschließend ein erneuter Lernvorgang durchgeführt wird.

2. Vorrichtung nach Anspruch 1, bei der das neurale Netzwerk Mittel zum Normieren der medizinischen Untersuchungsdaten derart enthält, daß Daten mit veränderlichen Werten zwischen 0 und 1 erhalten werden.

3. Vorrichtung nach Anspruch 1, bei der jeder Wert der medizinischen Untersuchungsdaten in mehrere verschiedene Bereiche unterteilt wird und das neurale Netzwerk (2) mit mehreren Eingabeeinheiten (601-672) ausgestattet ist, die in der Lage sind, diskrete Werte, die den unterschiedlichen Bereichen für jeden Wert der medizinischen Untersuchungsdaten zugeordnet sind, zu behandeln, wobei die mehreren Eingabeeinheiten (601-672) angeben, in welchen der unterschiedlichen Bereiche jeder Wert der medizinischen Untersuchungsdaten fällt.

4. Vorrichtung nach Anspruch 1, **gekennzeichnet** durch

eine Einrichtung (3) zum Speichern der medizinischen Untersuchungsdaten und der entsprechenden Lehrerdaten jedesmal dann, wenn die von dem neuralen Netzwerk vorgenommene Abschätzung an der Korrektureinrichtung korrigiert wird,

wobei das neurale Netzwerk (2) die menschlichen Beurteilungen der Notwendigkeit einer Neuuntersuchung erneut lernt, nachdem neue medizinische Untersuchungsdaten und entsprechende Lehrerdaten erneut in der Speichereinrichtung (3) abgespeichert sind.

5. Vorrichtung nach Anspruch 4, bei der die Speichereinrichtung (3) die medizinischen Untersuchungsdaten und die entsprechenden Lehrerdaten in der Reihenfolge der Erfassung der medizinischen Untersuchungsdaten speichert, wobei jüngste medizinische Untersuchungsdaten und entsprechende Lehrerdaten bezüglich derer die von dem neuralen Netzwerk (2) vorgenommene Abschätzung korrigiert wird, die ältesten medizinischen Untersuchungsdaten und entsprechende Lehrerdaten in der Speichereinrichtung (3) ersetzen.

6. Vorrichtung nach Anspruch 1, bei der die medizinischen Untersuchungsdaten mehrere Elemente umfassen und die medizinischen Untersuchungsdaten in Gruppen klassifiziert werden, in denen jede Gruppe eine unterschiedliche Kombination aus einer willkürlichen Anzahl von Elementen repräsentiert, und wobei das Lernen und Abschätzen seitens des neuralen Netzwerks bezüglich jeder Gruppe separat durchgeführt werden.

7. Vorrichtung nach Anspruch 2, bei der jeder Wert der medizinischen Untersuchungsdaten in mehrere verschiedene Bereiche unterteilt ist und das neurale Netzwerk (2) mit mehreren Eingabeeinheiten (601-672) ausgestattet ist, die in der Lage sind, sich ständig ändernde Werte, die den unterschiedlichen Bereichen für jeden Wert der medizinischen Untersuchungsdaten zugeordnet sind, zu behandeln, wobei die Normierung hinsichtlich jedes der unterschiedlichen Bereiche unabhängig voneinander stattfindet.

## Revendications

1. Un appareil pour analyser automatiquement la validité de données d'examen médical en évaluant la nécessité d'un réexamen, comprenant :

des moyens (1) pour collecter et examiner des données d'examen médical et pour générer des données supplémentaires, comprenant la présence ou l'absence de chaque élément d'un ensemble des données d'examen médical;

un réseau neuronal (2)

pour apprendre des jugements humains concernant la nécessité de réexamen, à partir d'ensembles collectés précédemment de données d'examen médical, accompagnés de données d'apprentissage correspondantes, ces données d'apprentissage indiquant si un réexamen était nécessaire pour les ensembles correspondants d'examen médical, grâce à quoi l'apprentissage au moyen de l'algorithme de propagation d'erreur en sens arrière est répété jusqu'à ce qu'une erreur devienne inférieure à un niveau déterminé; et après cet apprentissage initial

pour fournir une évaluation de la nécessité de réexamen pour chaque ensemble de données d'examen médical qui ont été collectées par les moyens de collecte; et

des moyens pour corriger l'évaluation de la nécessité de réexamen qui est faite par le réseau neuronal (2), si elle est considérée comme étant insuffisante, en comparaison avec un jugement humain, par la modification des données d'apprentissage, suivie par un réapprentissage.

2.    L'appareil de la revendication 1, dans lequel le réseau neuronal comprend des moyens pour normaliser des données d'examen médical, de façon à obtenir des données ayant des valeurs comprises entre 0 et 1.

3.    L'appareil de la revendication 1, dans lequel chaque valeur des données d'examen médical est répartie dans un ensemble de plages distinctes, et le réseau neuronal (2) est équipé d'un ensemble d'unités d'entrée (601-672) capables de travailler avec des valeurs discrètes qui sont affectées aux plages distinctes pour chaque valeur des données d'examen médical, l'ensemble d'unités d'entrée (601-672) indiquant dans quelle plage distincte particulière tombe chaque valeur des données d'examen médical.

4.    L'appareil de la revendication 1, comprenant en outre :

des moyens (3) pour mémoriser les données d'examen médical et les données d'apprentissage correspondantes, chaque fois que l'évaluation qui est faite par le réseau neuronal est corrigée dans les moyens de correction,

et dans lequel le réseau neuronal (2) apprend à nouveau les jugements humains concernant la nécessité de réexamen, après que de nouvelles données d'examen médical et des données d'apprentissage correspondantes ont été nouvellement mémorisées dans les moyens de mémorisation (3).

5.    L'appareil de la revendication 4, dans lequel les moyens de mémorisation (3) mémorisent les données d'examen médical et les données d'apprentissage correspondantes dans l'ordre d'acquisition des données d'examen médical, et dans lequel les données d'examen médical les plus récentes et les données d'apprentissage correspondantes en relation avec lesquelles l'évaluation faite par le réseau neuronal (2) est corrigée, remplacent les données d'examen médical les plus anciennes et les données d'apprentissage correspondantes, dans les moyens de mémorisation (3).

6.    L'appareil de la revendication 1, dans lequel les données d'examen médical comprennent un ensemble d'éléments, et les données d'examen médical sont classées en groupes, chaque groupe représentant une combinaison distincte d'un nombre arbitraire des éléments, et dans lequel l'apprentissage et l'évaluation qui sont effectués par le réseau neuronal sont accomplis séparément pour chaque groupe.

7.    L'appareil de la revendication 2, dans lequel chaque valeur des données d'examen médical est divisée en un ensemble de plages distinctes,le réseau neuronal (2) est équipé d'un ensemble d'unités d'entrée (601-672) capables de traiter des valeurs variant de façon continue qui sont attribuées aux plages distinctes, pour chaque valeur des données d'examen médical, et la normalisation est effectuée de façon indépendante par rapport à chacune des plages distinctes.

# FIG.1

FIG.2

EXAMINATION EQUIPMENT 1

NORMALIZED MEDICAL EXAMINATION DATA

2

ASSESSMENT

INPUT LAYER 6

INPUT UNIT 1 — 601

INPUT UNIT 2 — 602

INTERMEDIATE LAYER 7

OUTPUT LAYER 8

OUTPUT UNIT 801

TEACHER DATA

INPUT UNIT 71 — 671

INPUT UNIT 72 — 672

NEURAL NETWORK

EP 0 389 992 B1